(19) Europäisches Patentamt
European Patent Office
Office européen des brevets

(11) Publication number: **0 048 149**
**B1**

(12) # EUROPEAN PATENT SPECIFICATION

(45) Date of publication of patent specification: **30.11.83**

(51) Int. Cl.³: **C 07 G 11/00,**
A 61 K 35/36, A 61 K 35/56

(21) Application number: **81304186.0**

(22) Date of filing: **11.09.81**

(54) **Novel antibiotics, derivatives thereof, processes for their extraction, and compositions containing them.**

(30) Priority: **12.09.80 US 186932**
**18.12.80 US 217768**

(43) Date of publication of application:
**24.03.82 Bulletin 82/12**

(45) Publication of the grant of the patent:
**30.11.83 Bulletin 83/48**

(84) Designated Contracting States:
**BE CH DE FR GB IT LI NL SE**

(56) References cited:
**JOURNAL OF THE AMERICAN CHEMICAL SOCIETY, Vol. 103, No. 7, 8 April 1981 Columbus K.L. RINEHART JR. et al. "Structures of the Didemnins, Antiviral and Cytotoxic Depsipeptides from a Caribbean Tunicate" pages 1857-1859**

**Chemical Abstracts vol. 95, no. 5, 1981 Columbus, Ohio, USA K.L. RINEHART JR. et al. "Didemnins: antiviral and antitumor depsipeptides from a Caribbean tunicate" page 60, column 1, abstract no. 35522k**

(73) Proprietor: **University of Illinois Foundation**
**University of Illinois 346 Administration Building**
**Urbana, Illinois 61801 (US)**

(72) Inventor: **Rinehart, Kenneth Lloyd, Jr.**
**1306 South Carle Avenue**
**Urbana Illinois (US)**

(74) Representative: **Perry, Robert Edward et al,**
**GILL JENNINGS & EVERY 53-64 Chancery Lane**
**London WC2A 1HN (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 048 149 B1

# Novel antibiotics, derivatives thereof, processes for their extraction, and compositions containing them

This invention relates to novel antibiotics, to derivatives thereof, to processes for their extraction from organisms, and to compositions containing them.

The novel antibiotics have been named didemnin A, didemnin B, didemnin C, nordidemnin A, nordidemnin B and nordidemnin C. They have been extracted from a marine tunicate of the family Didemnidae tentatively identified as a *Trididemnum sp.* These marine tunicate organisms are in the suborder Aplousobranchia of the order Enterogona of the class Ascidiacea of the subphylum Urochordata of the phylum Chordata. They can be readily obtained in certain waters by scuba techniques at depths of 3 to 30 meters where they encrust, for example, rocks, sponges and gorgonians. They exist in colonies of sizes up to 1 meter in diameter and 1.25 cm in thickness. They vary in colour, depending on location, from green-white to purple-white to brown-white to orange-white.

Specific locations from which these organisms have been obtained are as follows:

(1) South-west side of Long Cay, Lighthouse Reef, Belize, 17° 11.8′ N by 87° 36.5′ W at a depth of 15 to 30 m,

(2) Rada el Covė, Isla San Andres, Colombia, 12° 31′ 46″ N by 81° 44′ 5″ W at 8 to 10 m;

(3) Palancar Reef, Isla de Cozumel, Mexico 20° 18.2′ N by 87° 2.5′ W at 18 to 30 m;

(4) On the west side of the southern tip of Turneffe Island, Belize, 17° 11.3′ N by 87° 55.6′ W at 15 to 23 m;

(5) Punta Oeste, Coxen's Hole Harbour, Isla Roatan, Honduras, 16° 15′ N by 86° 38′ W at 3 to 21 m;

(6) On the leeward side of the western-most Holandes Cay, Isla San Blas, Panama, 9° 35.6′ N by 78° 47′ W at about 18 m.

A variety of methods can be used to isolate and purify the didemnins and nordidemnins from samples of the tunicate organism, for example, solvent extraction, partition chromatography, silica gel chromatography, liquid-liquid distribution in a Craig apparatus, adsorption on resins, and crystallisation from solvents.

The preferred extraction method for the antibiotics of the invention comprises

(a) extracting a suitable marine tunicate with MeOH:toluene (3.1 v/v);

(b) subjecting the resulting extract to silica gel chromatography using the solvent system $CHCl_3$:MeOH in step gradient fashion; and

(c) recovering the antibiotic. For the nordidemnins, step (c) involves reverse phase high pressure liquid chromatography. The preferred marine tunicate is *Trididemnum sp.*

An example of the extraction process is as follows (all solvent proportions by volume);

## Example 1

Tunicate sample AHCE 614 was collected on the southwest side of Long Cay, Lighthouse Reef, Belize, 17° 11.8′ N by 87° 36.5′ W at a depth of 15 to 30 m. The sample was placed in isopropanol and stored at −10°C until it was extracted by the following procedure.

500 Grams of tunicate were homogenized in a Waring blender with a total of 2.4 liters of 3:1 MeOH:toluene and the residue was filtered with suction to give a dark olive-green solution. 500 ml of the aqueous isopropanol from the storage container was evaporated down to ~40 ml of a mostly aqueous, oily solution. This material was suspended in 400 ml of 3:1 methanol:toluene and combined with the 2.4 liters above.

The resulting dark green solution was partitioned versus 1400 ml of 1 *N* $NaNO_3$ solution to give an aqueous phase and a toluene phase. The aqueous phase was extracted with chloroform (1 × 500 ml, 1 × 300 ml, 2 × 250 ml and 1 × 100 ml) to give a cloudy grass green chloroform solution which was evaporated under reduced pressure, taken up in dry $CHCl_3$, and filtered to remove salt. Evaporation of the chloroform yielded 878 mg of a dark green flaky solid.

108 Mg of this material was loaded onto a silica gel (Brinkmann, particle size 0.05—0.2 mm) column of dimensions 1.7 cm × 46 cm, which was packed in chloroform. The column was eluted in step gradient fashion with 150 ml $CHCl_3$, 150 ml $CHCl_3$:MeOH, 99:1; 150 ml $CHCl_3$:MeOH, 97:3, and 250 ml $CHCl_3$:MeOH, 95:5. Three-ml fractions were collected, and the content of each fraction was evaluated by thin-layer chromatography developed with 9:1 $CHCl_3$:MeOH.

Relatively impure didemnin C (Rf 0.66—0.72) was obtained as an oil in trace amounts as the first major non-pigment to elute. It was contained in the last 30 ml of the 3% methanol in chloroform wash. Essentially pure didemnin C can be obtained by use of preparative thin-layer chromatography, as described for preparing didemnin A, below. Essentially pure didemnin B (Rf. 0.59—0.64) was obtained as a yellow-white amorphous solid (6.1 mg). In most cases, didemnin B was already pure enough (by TLC) for data accumulation and testing. Didemnin A (Rf 0.46—0.52) was obtained as a greenish-white solid (34.7 mg) containing substantial impurities of both higher and lower Rf. Didemnins B and A were contained in the first 100 ml of the 5% methanol in chloroform wash. When an essentially pure sample was desired (for mass spectroscopy, etc.), didemnin A was purified by preparative thin-layer chromatography on 0.25 mm TLC plates employing 9:1 $CHCl_3$:MeOH as the eluent. In many cases, the best

fractions of didemnin A collected from later columns had fewer impurities, which allowed immediate use for reactions and spectral data accumulation.

Nordidemnins (A, B and C) can be separated from the didemnins and isolated in essentially pure form by reverse phase high pressure liquid chromatography, monitoring the N,O-dimethyltyrosine chromophore by ultraviolet spectroscopy at 275 nm. Suitable solvent systems for these separations include combinations of methanol, water and triethylamine; methanol, water and 2-propanol; or ethanol, water and 2-propanol.

Since the antibiotics of the invention are weakly basic, they form salts with mineral acids such as HCl, $H_2SO_4$ and $H_3PO_4$. Such salts can be prepared by suspending the antibiotic in water, adding a dilute acid until the pH of the solution is, for example, 3 to 4, and freeze-drying the solution to provide a dried residue of the salt. Salts of the antibiotics can be used for the same biological purposes as the parent compounds.

The antibiotics have free amino and hydroxyl groups available for derivatisation. Thus, acyl amides and esters can be prepared by methods well known to those skilled in the art. The acyl derivatives can be used for the same biological purposes as the parent compounds.

An acylate of an antibiotic of the present invention is one in which the acyl radical is ($C_{1-8}$ alkoxy)carbonyl or optionally substituted hydrocarbon $C_{2-18}$ acyl in which any substituent is selected from halogen, nitro, hydroxy, amino, cyano, thiocyano and $C_{1-8}$ alkoxy. The acid from which the acylate is derived may be selected from, for example, (a) saturated or unsaturated, straight or branched chain aliphatic carboxylic acids, for example, acetic, propionic, butyric, isobutyric, *tert*-butylacetic, valeric, isovaleric, caproic, caprylic, decanoic, dodecanoic, lauric, tridecanoic, myristic, pentadecanoic, palmitic, margaric, stearic, acylic, crotonic, undecylenic, oleic, hexynoic, heptynoic or octynoic acid; (b) saturated or unsaturated, alicyclic carboxylic acids, for example, cyclobutanecarboxylic acid, cyclopentanecarboxylic acid, cyclopentenecarboxylic acid, methylcyclopentenecarboxylic acid, cyclohexanecarboxylic acid, dimethylcyclohexanecarboxylic acid or dipropylcyclohexanecarboxylic acid; (c) saturated or unsaturated, alicyclic aliphatic carboxylic acids, for example, cyclopentaneacetic acid, cyclopentanepropionic acid, cyclohexaneacetic acid, cyclohexanebutyric acid, or methylcyclohexaneacetic acids; (d) aromatic carboxylic acids, for example, benzoic acid, toluic acid, naphthoic acid, ethylbenzoic acid, isobutylbenzoic acid, methylbutylbenzoic acid, and the like; and (e) aromatic-aliphatic carboxylic acids, for example, phenylacetic acid, phenylpropionic acid, phenylvaleric acid, cinnamic acid, phenylpropiolic acid or naphthylacetic acid.

Suitable halo-, nitro-, hydroxy-, keto-, amino-, cyano-, thiocyano-, and alkoxyhydrocarbon carboxylic acids include hydrocarboncarboxylic acids as given above which are substituted by one or more of halogen, nitro, hydroxy, keto, amino, cyano, thiocyano and alkoxy, advantageously alkoxy of not more than six carbon atoms, for example, methoxy, ethoxy, propoxy, butoxy, amyloxy, hexyloxy, and isomeric forms thereof. Examples of such substituted hydrocarbon carboxylic acids are:

mono-, di-, and trichloroacetic acid;
α- and β-chloropropionic acid;
α- and γ-bromobutyric acid;
α- and δ-iodovaleric acid;
mevalonic acid;
2- and 4-chlorocyclohexanecarboxylic acid;
shikimic acid;
2-nitro-1-methyl-cyclobutanecarboxylic acid;
1,2,3,4,5,6-hexachlorocyclohexanecarboxylic acid;
3-bromo-2-methylcyclohexanecarboxylic acid;
4- and 5-bromo-2-methylcyclohexanecarboxylic acid;
5- and 6-bromo-2-methylcyclohexanecarboxylic acid;
2,3-dibromo-2-methylcyclohexanecarboxylic acid;
2,5-dibromo-2-methylcyclohexanecarboxylic acid;
4,5-dibromo-2-methylcyclohexanecarboxylic acid;
5,6-dibromo-2-methylcyclohexanecarboxylic acid;
3-bromo-3-methylcyclohexanecarboxylic acid;
6-bromo-3-methylcyclohexanecarboxylic acid;
1,6-dibromo-3-methylcyclohexanecarboxylic acid;
2-bromo-4-methylcyclohexanecarboxylic acid;
1,2-dibromo-4-methylcyclohexanecarboxylic acid;
3-bromo-2,2,3-trimethylcyclopentanecarboxylic acid;
1-bromo-3,5-dimethylcyclohexanecarboxylic acid;
homogentisic acid, o-, m-, and p-chlorobenzoic acid;
anisic acid;
salicylic acid;
p-hydroxybenzoic acid;
β-resorcylic acid;
gallic acid;

veratric acid;
trimethoxybenzoic acid;
trimethoxycinnamic acid;
4,4′-dichlorobenzilic acid;
o-, m-, and p-nitrobenzoic acid;
cyanoacetic acid;
3,4- and 3,5-dinitrobenzoic acid;
2,4,6-trinitrobenzoic acid;
thiocyanoacetic acid;
cyanopropionic acid;
lactic acid;
ethoxyformic acid (ethyl hydrogen carbonate);
malic acid;
citric acid;
isocitric acid;
6-methylsalicylic acid;
mandelic acid;
levulinic acid;
pyruvic acid;
glycine;
alanine;
valine;
isoleucine;
leucine;
phenylalanine;
proline;
serine;
threonine;
tyrosine;
hydroxyproline;
ornithine;
lysine;
arginine;
histidine;
hydroxylysine;
phenylglycine;
p-aminobenzoic acid;
m-aminobenzoic acid;
anthranilic acid;
aspartic acid;
glutamic acid;
aminoadipic acid;
glutamine; and
asparagine.

The characteristics of the novel antibiotics are as follows:

Didemnins A, B and C are soluble in methanol, ethanol, isopropanol, dioxane, ethyl acetate, and chloroform. They are only sparingly soluble in toluene and insoluble in water. Nordidemnins A, B and C have a similar solubility pattern.

The didemnin samples were hydrolyzed in 6 *N* HCl at 110°C for 24 hours. The resulting amino acids were identified by field desorption mass spectrometry (FDMS) of the mixture, as well as by gas chromatography (GC)/MS of the amino acids' trifluoroacetyl *n*-butyl ester derivatives. They were also quantitated by GC, and their identities confirmed by coinjection with derivatives of authentic samples.

Didemnin A contains a mole each of leucine; N-methylleucine, threonine; proline; N,O-dimethyltryosine; and statine [see H. Morishima *et. al.,* Journal of Antibiotics *23*:263 (1970) for a description of statine (from pepstatin)]. Statine was assigned as the *threo* isomer by its co-elution with the synthetic *R,S*-isomer, while gas chromatography on an optically active column indicated Leu, MeLeu, and $Me_2$Tyr to have the L-configuration.

Didemnin B contains a mole of each of the above six amino acids plus a mole of lactic acid and an additional mole of proline. Didemnin C contains a mole of each of the above six amino acids plus a mole of lactic acid. In addition to the above amino acids, each didemnin contains a hydroxy-isovalerylpropionyl group.

The order of linkage of these seven units was established by the fragment ions identified in high resolution electron impact mass spectra.

Each of the didemnins (A, B, C) is accompanied by a minor but varying amount of a homologue. The nature of the homology was clarified by the observation that GC traces of the derivatized amino

acids always contain minor amounts of an amino acid identified by GC/MS as a homologue of statine, for which we propose the name norstatine [$(CH_3)_2CHCHNH_2CHOHCH_2COOH$]. The homologous peptides have been designated as nordidemnins A, B and C, in which norstatine replaces the statine unit of each didemnin.

*Mass Spectra*

Didemnin A has the following mass spectral peaks: a molecular ion at 942.5678 ($C_{49}H_{78}N_6O_{12}$) and fragments at *m/z* 886, 843, 800, 756, 611, 583, 557, 523, 501, 499, 480, 455, 401, 383, 356, 313, 310, 298, 288, 262, 210, 183, 178, 154, 139, 134, 128, 122, 121, 100.

The field desorption mass spectrum of didemnin B gives an M + H ion at 1112.6442 ($C_{57}H_{90}N_7O_{15}$). The electron impact mass spectrum of didemnin B contains ions at *m/z* 942, 924, 913, 886, 844, 843, 797, 756, 611, 593, 557, 523, 425, 396, 383, 313, 307, 288, 262, 224, 210, 183, 154, 121, 100.

The field desorption mass spectrum of didemnin C contains an M+H ion at 1014.5873 ($C_{52}H_{82}N_6O_{14}$). In addition, the mass spectrum of didemnin C obtained by electron impact gives ion at *m/z* 958, 887, 859, 842, 693, 675, 578, 547, 536, 494, 368, 283, 262, 237, 224, 200, 172, 154, 121, 100. The minor homolog of didemnin C gives an M+H ion at 1000.5714 ($C_{51}H_{80}N_6O_{14}$). Its electron impact mass spectrum contains peaks at *m/z* 944, 873, 845 and 828 indicative of the homology.

*NMR Spectra*

Didemnin A had the following proton NMR peaks, in ppm from tetramethylsilane: 8.3, 7.8, 7.5, 7.1, 6.9, 5.23, 5.0, 4.9, 4.8, 4.6, 4.2, 4.1, 4.0, 3.8, 3.7, 3.6, 3.4, 3.2, 3.1, 2.9, 2.6 (singlet methyl), 2.6 (doublet of doublets), 2.4 (singlet methyl), 2.4 (multiplet), 2.1, 1.8, 1.6, 1.4, 1.35, 1.30, 1.2, 0.9 (several overlapping methyl doublets).

Didemnin B had the following proton NMR peaks, in ppm from tetramethysilane: 8.0, 7.8, 7.4, 7.2, 7.0, 5.5, 5.4, 5.3, 4.9, 4.8, 4.6, 4.5, 4.4, 4.2, 3.9, 3.8, 3.5, 3.4, 3.3 (doublet), 3.3 (singlet methyl), 3.1, 2.8, 2.7 (singlet methyl), 2.5, 2.3, 2.1, 1.9, 1.7, 1.5, 1.4, 1.3, 1.0 (several overlapping methyl doublets).

Didemnin C had the following proton NMR peaks, in ppm from tetramethylsilane: 7.7, 7.4, 7.2, 7.0, 5.2, 5.0, 4.8, 4.5, 4.2, 4.0, 3.7, 3.5, 3.3, 3.1, 3.0, 2.9, 2.8 (singlet methyl), 2.4 (singlet methyl), 2.3, 2.2, 2.1, 1.8, 1.5, 1.4—1.25, 1.1—0.8 (several overlapping methyls).

Didemnin A had the following $^{13}C$—NMR signals, relative to tetramethylsilane in $CDCl_3$: 205.1, 175.3, 172.3, 171.4, 170.4, 169.9, 169.6, 168.6, 158.7, 130.4, (2 carbons), 129.9, 11.4 (2 carbons), 81.5, 71.1, 67.6, 66.2, 63.2, 57.4, 55.4, 55.3, 54.7, 49.9, 49.5, 47.1, 42.5, 41.3, 38.6 (2 carbons), 35.4, 34.2, 34.0, 31.2, 27.9, 27.0, 25.1 (2 carbons), 24.9, 23.7, 22.9, 22.3, 21.0, 18.7, 16.9, 15.4, 14.9 (2 carbons), 11.7.

Didemnin B has the following $^{13}C$—NMR signals relative to tetramethyl silane in $CDCl_3$: 204.9, 174.0, 172.9, 172.4, 171.8, 171.3, 170.6, 169.7, 169.4, 168.4, 158.7, 130.3 (2 carbons) 130.1, 114.2 (2 carbons), 81.5, 70.5, 68.0, 66.5, 66.0, 57.7, 57.2, 56.7, 55.5, 55.3, 54.9, 49.6 (2 carbons), 47.0 (2 carbons), 41.4, 38.9, 38.7, 36.2, 34.0 (2 carbons), 31.3 (2 carbons), 28.4, 27.9, 27.2, 26.0, 24.9 (3 carbons) 23.8, 23.4, 21.4, 21.0, 20.3, 18.6, 16.9, 16.3, 15.2, 14.7, 11.7.

*Infrared Spectra*

The didemnins were dissolved in chloroform and examined in a Beckman IR—12 double beam spectro-photometer vs. a chloroform reference.

The spectrum of didemnin A is shown in Figure 1. Peaks are observed at the following wavelengths:

| Band Frequency (Wave Number $cm^{-1}$) | Intensity (%T) |
|---|---|
| 3680 | 84 |
| 3600 | 82 sh |
| 3520 | 70 |
| 3340 | 54 |
| 3020 | 57 |
| 2970 | 39 |
| 2940 | 51 |

| Band Frequency (Wave Number $cm^{-1}$) | Intensity (%T) |
|---|---|
| 2880 | 60 |
| 2810 | 78 |
| 2460 | 82 |
| 1725 | 27.5 |
| 1650 | 23 |
| 1630 | 17 |
| 1605 | 64 |
| 1540 | 48 |
| 1505 | 31 |
| 1455 | 45 |
| 1445 | 43 |
| 1405 | 61 |
| 1380 | 59 |
| 1360 | 60 |
| 1335 | 60.5 |
| 1325 | 64 |
| 1310 | 60 sh |
| 1295 | 53 |
| 1265 | 51 |
| 1240 | 43 |
| 1195 | 63 sh |
| 1160 | 40 |
| 1110 | 62 |
| 1100 | 63.5 |
| 1080 | 66 sh |
| 1065 | 60 |
| 1030 | 71 |
| 1000 | 68 |
| 965 | 73 |
| 940 | 75 |
| 920 | 76 |

| Band Frequency (Wave Number $cm^{-1}$) | Intensity (%T) |
|---|---|
| 900 | 78 |
| 825 | 72 |
| 660 | 65 |
| 620 | 79 |

sh = shoulder

The spectrum of didemnin B is shown in Figure 2. Peaks are observed at the following wavelengths:

| Band Frequency (Wave Number $cm^{-1}$) | Intensity (%T) |
|---|---|
| 3680 | 80 |
| 3600 | 79 |
| 3340 | 52.5 |
| 3020 | 51.5 |
| 2970 | 38 |
| 2940 | 47 |
| 2880 | 58 |
| 2460 | 81 |
| 1725 | 28 |
| 1650 | 17 |
| 1640 | 13 |
| 1630 | 15 sh |
| 1605 | 51 sh |
| 1540 | 44 |
| 1514 | 38.5 |
| 1465 | 41 |
| 1455 | 40 |
| 1415 | 52 |
| 1390 | 52.5 |
| 1370 | 52 |
| 1345 | 57.5 |
| 1300 | 51 |
| 1270 | 46 |

| Band Frequency (Wave Number $cm^{-1}$) | Intensity (%T) |
|---|---|
| 1250 | 40.5 |
| 1170 | 42 |
| 1120 | 56 |
| 1105 | 59 |
| 1075 | 56 |
| 1040 | 63.5 |
| 1000 | 70 |
| 965 | 73.5 |
| 940 | 77 |
| 910 | 76.5 |
| 900 | 78 |
| 865 | 78 |
| 830 | 76 |
| 660 | 75 |
| 615 | 80 |

sh = shoulder

Antiviral Acitivity

The antiviral activities of didemnins A, B and C are shown in the following table. The test method is as follows:

Costar 96 well trays were seeded with 0.2 ml cell suspension and incubated at 37° for 24 hours. The medium was removed and the wells were treated with serial 2-fold dilutions of drug (150—1.5 μg/ml) in 0.05 ml. Diluted virus (0.05 ml) or medium (BME—3% fbs) was added to each well and the cultures were returned to 37°. After overnight incubation, the cells were stained with aqueous crystal violet (0.5%), and washed thoroughly. Drug concentrations resulting in 50% cell destruction ($ID_{50}$) were determined visually for those cultures infected with virus (antiviral) as well as those cultures serving as toxicity controls. Ratios are the cytotoxic concentration ÷ antiviral concentration.

The cells used for the herpes simplex virus (HSV) type 1 and type 2 were Vero. ML cells were used for the assays with coxsackie (COX) A21 and equine rhinovirus (ER).

| RNA Viruses | ID$_{50}$ A | B | C |
|---|---|---|---|
| Toxicity-MI cells | 25 | <1.5 | 6 |
| Antiviral-cox virus | 1.5 | <1.5 | <1.5 |
| Ratio | 17 | | >4 |
| Toxicity-ML cells | 25 | <1.5 | 6 |
| Antiviral-ER virus | 1.5 | <1.5 | <1.5 |
| Ratio | 17 | | >4 |
| DNA Viruses | | | |
| Toxicity-Vero cells | 50 | 12 | 50 |
| Antiviral-HSV—1 virus | 3 | 1.5 | <1.5 |
| Ratio | 17 | 8 | >33 |
| Toxicity-Vero cells | 50 | 12 | 50 |
| Antiviral-HSV—2 virus | 1.5 | 1.5 | <1.5 |
| Ratio | 33 | 8 | >33 |

Antileukemia Activity

Didemnins A and B inhibit the growth of L1210 mouse leukemia cells *in vitro* as shown in the following table. The L1210 tube dilution assay is described in detail in a publication by L. H. Li, *et al.,* Cancer Research *39:*4816 (1979). ID$_{50}$ and ID$_{90}$ refer to the concentration of didemnins needed to inhibit cell growth by 50 and 90 percent, respectively.

L1210 Tube Dilution Assay

| | ID$_{50}$ μg/ml | ID$_{90}$ μg/ml |
|---|---|---|
| Didemnin A | 0.019 | 0.058 |
| Didemnin B | 0.0018 | 0.0059 |

Didemnins A and B were also active *in vivo* against P388 leukemia in mice. The P388 mouse leukemia test is described in detail in a publication by G. L. Neil, et al., Cancer Treatment Reports *63,* 1971—1978 (1979). The results of two P388 mouse leukemia tests using different dosage schedules is shown below.

In Vivo Testing of Didemnins Against P388 Leukemia

| Compound[a] | Dose ($\mu$g/kg/ injection) | Median Survival Time[b] (day) | T/C (%) | Weight Change (g) |
|---|---|---|---|---|
| Didemnin A | 63 | 10.7 | 104 | +0.9 |
| | 125 | 11.9 | 115 | +1.4 |
| | 250 | 12.2 | 119 | +1.0 |
| | 500 | 13.2 | 129 | +0.9 |
| | 1000 | 16.2 | 158 | +0.3 |
| | 2000 | 16.0 | 155 | −0.2 |
| Didemnin B | 63 | 14.8 | 143 | +0.1 |
| | 125 | 14.0 | 136 | −0.6 |
| | 250 | 14.2 | 138 | −2.2 |
| | 500 | T[c] | T | −3.4 |
| | 1000 | T | T | T |
| | 2000 | T | T | T |

[a] Schedule of Injection: daily intraperitoneal injection for 9 days following tumor implantation.
[b] Median survival time of control animals = 10.3 days.
[c] Toxic.

| Compound[a] | Dose ($\mu$g/kg/ injection) | Median Survival Time[b] (day) | T/C (%) | Weight Change (g) |
|---|---|---|---|---|
| Didemnin A | 250 | 11.1 | 109 | +1.7 |
| | 500 | 12.1 | 118 | +1.6 |
| | 1000 | 12.1 | 118 | +1.8 |
| | 2000 | 12.4 | 121 | +2.0 |
| | 4000 | 13.3 | 130 | +1.3 |
| | 8000 | 14.3 | 140 | +0.8 |
| Didemnin B | 30 | 12.8 | 125 | +1.6 |
| | 60 | 14.3 | 140 | +1.2 |
| | 120 | 15.0 | 147 | +0.2 |
| | 250 | 16.0 | 157 | −0.5 |
| | 500 | 18.0 | 176 | −1.7 |
| | 1000 | 20.3 | 199 | −3.8 |

[a] Schedule of injection: Intraperitoneal injection on Day 1, 5, and 9, following Tumor inoculation.
[b] Median survival time of Control (no drug) animals: 10.2 days.

Nordidemnins A, B and C give molecular ions at *m/z* 928, 1097 and 1000, respectively.

The novel antibiotics can have pharmaceutical utility. A pharmaceutical composition according to the invention comprises an antibiotic of the invention, or a salt or acrylate thereof, in association with a physiologically acceptable excipient.

The antibiotics may have activity against DNA viruses, for example, herpes simplex virus types 1 and 2, and vaccinia virus; RNA viruses, for example, coxsackie virus and equine rhinovirus; and R399 leukemia in mice. Thus, these antibiotics may be useful for the treatment of infections in humans, animals and plants caused by these viruses and other DNA and RNA viruses; they may also have utility in the treatment of neoplastic diseases in animals and humans. Didemnin A and didemnin B also inhibit L1210 mouse leukemia cells *in vitro*. Acid addition salts and acyl derivatives can be made and used for the same biological purposes as the parent compounds.

The administration of didemnins A, B or C and nordidemnins A, B or C is useful prophylactically and therapeutically for treating neoplastic diseases and viral infections. For example, pharmaceutical compositions containing the active ingredients are useful in prophylactic or therapeutic treatment of humans and animals infected or likely to be infected with viruses, e.g., hepatitis virus, rubella, rubeola, influenza, encephalitis viruses (i.e., arboviruses such as western or eastern equine encephalitis virus, Semliki Forest virus), herpes viruses (types 1 or 2 herpes simplex virus, cytomegalovirus, varicella-zoster and infectious bovine rhinotracheitis virus), rabies enteroviruses (picornaviruses, echoviruses, coxsackie viruses), parainfluenza viruses, respiratory syncytial virus, sendai virus, poliomyelitis viruses, yellow fever, Epstein-Barr virus (infectious mononucleuosis), small pox, Dengue virus, common cold virus (rhinoviruses, coronaviruses, etc.), adenoviruses, polyomaviruses, papovaviruses, RNA-tumor viruses (e.g., feline leukemia virus, avian leukosis virus, avian sarcoma viruses), B virus, aleutians disease of mink, arena viruses, blue tongue virus of sheep, bovine viral diarrhea-mucosal disease virus, canine distemper virus, canine hepatitis virus, canine herpesvirus, equine abortion virus, infectious equine anemia virus, fowl pox virus, hog cholera virus, Marek's disease, mink enteritis virus, Newcastle disease virus, porcine enterovirus, pseudorabies virus, foot and mouth disease virus, reoviruses, and all other viruses or diseases of viral origin (for example, slowly progressing diseases that may be of viral origin such as multiplesclerosis) that are sensitive to the antiviral action of the didemnins or nordidemnins.

The compounds of the subject invention also can be used to treat animals or humans hosting a neoplastic disease, for example, acute myelocytic leukemia, acute lymphocytic leukemia, malignant melanoma, adenocarcinoma of lung, neroblastoma, small cell carcinoma of lung, breast carcinoma, colon carcinoma, ovarian carcinoma or bladder carcinoma.

The dosage administered will be dependent upon the identity of the viral infection of neoplastic disease, the type of host involved, its age, health, weight, kind of concurrent treatment, if any, frequency of treatment and therapeutic ratio.

Illustratively, dosage levels of the administered active ingredients can be: intravenous, 0.01 to 20 mg/kg, intraperitoneal, 0.01 to 100 mg/kg; subcutaneous, 0.01 to 100 mg/kg; intramuscular 0.01 to 100 mg/kg; orally, 0.01 to 200 mg/kg, and preferably about 1 to 100 mg/kg; intranasal instillation, 0.01 to 20 mg/kg; and aerosol, 0.01 to 20 mg/kg of animal (body) weight.

Expressed in terms of concentration, an active ingredient can be present in the compositions of the present invention for localized use about the cutis, intranasally, pharyngolaryngeally, bronchially, broncholially, intravaginally, rectally, or ocularly in a concentration of from 0.01 to 50% w/w of the composition; preferably 1 to 20% w/w of the composition; and for parenteral use in a concentration of from 0.05 to 50% w/v of the composition and preferably from 5 to 20% w/v.

The compositions of the present invention are preferably presented for administration to humans and animals in unit dosage forms, such as tablets, capsules, pills or suppositories, or as powders granules, sterile parenteral solutions or suspensions, sterile non-parenteral solutions or suspensions, or oral solutions or suspensions, containing suitable quantities of an active ingredient.

For oral administration, either solid or fluid dosage forms can be prepared.

Powders are prepared quite simply by comminuting the active ingredient to a suitably fine size and mixing with a similarly comminuted diluent. The diluent can be an edible carbohydrate material such as lactose or starch. Advantageously, a sweetening agent or sugar is present as well as a flavoring oil.

Capsules are produced by preparing a powder mixture as hereinbefore described and filling into formed gelatin sheaths. Advantageously, as in adjuvant to the filling operation, a lubricant such as a talc, magnesium stearate or calcium stearate is added to the powder mixture before the filling operation.

Soft gelatin capsules are prepared by machine encapsulation of a slurry of active ingredients with an acceptable vegetable oil, light liquid petrolatum or other inert oil or triglyceride.

Tablets are made by preparing a powder mixture, granulating or slugging, adding a lubricant and pressing into tablets. The powder mixture is prepared by mixing an active ingredient, suitably comminuted, with a diluent or base such as starch, lactose, kaolin or dicalcium phosphate. The powder mixture can be granulated by wetting with a binder such as corn syrup, gelatin solution, methylcellulose solution or acacia mucilage and forcing through a screen. As an alternative to granulating, the powder

mixture can be slugged, i.e., run through the tablet machine and the resulting imperfectly formed tablets broken into pieces (slugs). The slugs can be lubricated to prevent sticking to the tablet-forming dies by means of the addition of stearic acid, a steraric salt, talc or mineral oil. The lubricated mixture is then compressed into tablets.

Advantageously the tablet can be provided with a protective coating consisting of a sealing coat or enteric coat of shellac, a coating of sugar and methylcellulose and polish coating of carnauba wax.

Fluid dosage forms for oral administration such as syrups, elixirs and suspensions can be prepared wherein each measure of composition contains a predetermined amount of active ingredient for administration. The water-soluble forms can be dissolved in an aqueous vehicle together with sugar, flavoring agents and preservatives to form a syrup. An elixir is prepared by using a hydroalcoholic vehicle with suitable sweeteners together with a flavoring agent. Suspensions can be prepared of the insoluble forms with a suitable vehicle with the aid of a suspending agent such as acacia, tragacanth or methylcellulose.

For parenteral administration, fluid dosage forms are prepared utilizing an active ingredient and a sterile vehicle, water being preferred. The active ingredient, depending on the form and concentration used, can be either suspended or dissolved in the vehicle. In preparing solutions the water-soluble active ingredient can be dissolved in water for injection and filter sterilized before filling into a suitable vial or ampule and sealing. Advantageously, adjuvants such as a local anesthetic, preservative and buffering agents can be dissolved in the vehicle. Parenteral suspensions are prepared in substantially the same manner except that an active ingredient is suspended in the vehicle instead of being dissolved and sterilization cannot be accomplished by filtration. The active ingredient can be sterilized by exposure to ethylene oxide before suspending in the sterile vehicle. Advantageously, a surfactant or wetting agent is included in the composition to facilitate uniform distribution of the active ingredient.

In addition to oral and parenteral administration, the rectal and vaginal routes can be utilized. An active ingredient can be administered by means of a suppository. A vehicle which has a melting point at about body temperature or one that is readily soluble can be utilized. For example, cocoa butter and various polyethylene glycols (Carbowaxes) can serve as the vehicle.

For intranasal instillation, fluid dosage forms are prepared utilizing an active ingredient and a suitable pharmaceutical vehicle, water being preferred, or by dry powder for insufflation.

The active ingredients can also be admixed in animal feed. The active ingredients can conveniently be prepared in the form of a food premix. The food premix can comprise an active ingredient in admixture with an edible pharmaceutical diluent such as starch, oatmeal, flour, calcium carbonate, talc, dried fish meal or similar nontoxic, orally acceptable pharmaceutical diluents. The prepared premix is then conveniently added to the regular feed.

For use as aerosols, the active ingredients can be packaged in a pressurized aerosol container together with a gaseous or liquefied propellant, for example, dichlorodifluoromethane, carbon dioxide, nitrogen or propane, with the usual adjuvants such as cosolvents and wetting agents, as may be necessary or desirable.

The term "unit dosage form" refers to physically discrete units suitable as unitary dosages for human and animal subjects, each unit containing a predetermined quantity of active material calculated to produce the desired therapeutic effect in association with the required pharmaceutical diluent, carrier or vehicle.

The active ingredients to be employed as antiviral or antineoplastic agents can be easily prepared in dosage forms with the employement of pharmaceutical materials which themselves are available in the art and can be prepared by established procedures.

The following Examples are illustrative of compositions of the present invention. Any reference to a didemnin may be taken to refer also to any nordidemnin.

Example 2
Hard Gelatin Capsules

One thousand two-piece hard gelatin capsules for oral use, each capsule containing 100 mg of a didemnin or a nordidemnin, are prepared from the following types and amounts of ingredients:

| | |
|---|---|
| A didemnin, micronized | 100 g |
| Lactose | 100 g |
| Corn Starch | 20 g |
| Talc | 20 g |
| Magnesium stearate | 2 g |

The didemnin, finely divided by means of an air micronizer, is added to the other finely powdered ingredients, mixed thoroughly and then encapsulated in the usual manner.

The foregoing capsules are useful for preventing or treating viral infection or for treating a neoplastic disease by the oral administration of one or two capsules one to four times a day.

Using the procedure above, capsules are similarly prepared containing didemnin or a nordidemnin in 50, 250 and 500 mg amounts by substituting 50 g, 250 g and 500 g of didemnin for the 100 g used above.

Example 3
Soft Gelatin Capsules

One-piece soft gelatin capsules for oral use, each containing 250 mg of a didemnin or a nordidemnin (finely divided by means of an air micronizer), are prepared by first suspending the compound in 0.5 ml of corn oil to render the material capsulatable and then capsulating in the above manner.

The foregoing capsules are useful for preventing or treating viral infection or for treating a neoplastic disease by the oral administration of one or two capsules one to four times a day.

Example 4
Tablets

One thousand tablets, each containing 500 mg of a didemnin or a nordidemnin are prepared from the following types and amounts of ingredients:

| | |
|---|---|
| A didemnin, micronized | 500 g |
| Lactose | 75 g |
| Corn starch | 50 g |
| Magnesium stearate | 4 g |
| Light liquid petrolatum | 5 g |

The didemnin, finely divided by means of an air micronizer, is added to the other ingredients and then thoroughly mixed and slugged. The slugs are broken down by forcing through a Number Sixteen screen. The resulting granules are then compressed into tablets, each tablet containing 500 mg of the didemnin.

The foregoing tablets are useful for preventing or treating viral infection or for treating a neoplastic disease by the oral administration of one or two tablets one to four times a day.

Using the procedure above, tablets are similarly prepared containing a didemnin or a nordidemnin in 250 mg and 100 mg amounts by substituting 250 g and 10 g of a didemnin for the 500 g used above.

Example 5
Oral Suspension

One thousand ml of an aqueous suspension for oral use, containing in each teaspoonful (5 ml) dose, 500 mg of a didemnin or a nordidemnin, is prepared from the following types and amounts of ingredients:

| | |
|---|---|
| A didemnin, micronized | 100 g |
| Citric acid | 2 g |
| Benzoic acid | 1 g |
| Sucrose | 700 g |
| Tragacanth | 5 g |
| Lemon Oil | 2 g |

Deionized water, q.s. 1000 ml.

The citric acid, benzoic acid, sucrose, tragacanth and lemon oil are dispersed in sufficient water to make 850 ml of suspension. The didemnin, finely divided by means of an air micronizer, is stirred into the syrup until uniformly distributed. Sufficient water is added to make 1000 ml.

The composition so prepared is useful for preventing or treating viral infection or for treating a neoplastic disease at a dose of 1 tablespoonful (15 ml) three times a day.

Example 6

A sterile aqueous suspension for parenteral injection, containing 300 mg/ml of a didemnin, is prepared from the following types and amounts of ingredients:

| | |
|---|---|
| A didemnin, micronized | 300 g |
| Polysorbate 80 | 5 g |
| Methylparaben | 2.5 g |
| Propylparaben | 0.17 g |
| Water for injection, q.s. 1000 ml. | |

All the ingredients, except the didemnin, are dissolved in the water and the solution sterilized by filtration. To the sterile solution is added the sterilized didemnin, finely divided by means of an air micronizer, and the final suspension is filled into sterile vials and the vials sealed.

The composition so prepared is useful for preventing or treating viral infection or for treating a neoplastic disease at a dose of 1 milliliter three times a day.

Example 7
Suppository, Rectal and Vaginal

One thousand suppositories, each weighing 2.5 gm and containing 150 mg of a didemnin or a nordidemnin, are prepared from the following types and amounts of ingredients:

| | |
|---|---|
| A didemnin, micronized | 150 g |
| Propylene glycol | 150 g |
| Polyethylene glycol 4000, q.s. | 2,500 g |

The didemnin is finely divided by means of an air micronizer and added to the propylene glycol and the mixture passed through a colloid mill until uniformly dispersed. The polyethylene glycol is melted and the propylene glycol dispersion added slowly with stirring. The suspension is poured into unchilled molds at 40°C. The composition is allowed to cool and solidify and then removed from the mold and each suppository foil wrapped.

The foregoing suppositories are inserted rectally or vaginally for preventing or treating viral infection, or for treating a neoplastic disease.

Example 8
Intranasal Suspension

One thousand ml of a sterile aqueous suspension for intranasal instillation, containing in each ml 150 mg of a didemnin or a nordidemnin, is prepared from the following types and amounts of ingredients:

| | |
|---|---|
| A didemnin, micronized | 150 g |
| Polysorbate 80 | 5 g |
| Methylparaben | 2.5 g |
| Propylparaben | 0.17 g |
| Deionized water, q.s. 1000 ml. | |

All the ingredients, except the didemnin, are dissolved in the water and the solution sterilized by filtration. To the sterile solution is added the sterilized didemnin, finely divided by means of an air micronizer, and the final suspension is aseptically filled into sterile containers.

The composition so prepared is useful for preventing or treating viral infection, or for treating a neoplastic disease, by intranasal instillation of 0.2 to 0.5 ml given one to four times per day.

Example 9
Animal Feed

One thousand grams of feed premix is prepared from the following types and amounts of ingredients:

| | |
|---|---|
| A didemnin | 20 g |
| Soybean meal | 400 g |
| Fish meal | 400 g |
| Wheat germ oil | 50 g |
| Sorghum molasses | 130 g |

The ingredients are mixed together and pressed into pellets.

The premix can be fed directly to laboratory animals, i.e., rats and mice, for preventing or treating viral infection.

For larger animals the premix can be added to the animal's regular feed in an amount calculated to give the desired dose of didemnin. For example, one part of premix is added to 2.5 parts of a cat's regular feed to provide the desired dose of 200 mg/kg/day for a cat of 2.5 kg.

An active ingredient can also be used, as shown in Examples 10—13, in undiluted pure form for use locally about the cutis, intranasally, pharyngolaryngeally, bronchially, broncholially or orally.

Example 10
Powder

Five hundred grams of a didemnin or a nordidemnin in bulk form is finely divided by means of an air micronizer. The micronized powder is placed in a shaker-type container.

The foregoing composition is useful for preventing or treating viral infection, or for treating a neoplastic disease, at localized sites by applying the powder one to four times per day.

Example 11
Oral Powder

One thousand grams of a didemnin or a nordidemnin in bulk form is finely divided by means of an air micronizer. The micronized powder is divided into individual doses of 250 mg and packaged.

The foregoing powders are useful for preventing or treating viral infection, or for treating a neoplastic disease, by the oral administration of one or two powders suspended in a glass of water, one to four times per day.

Example 12
Insufflation

One thousands grams of a didemnin or a nordidemnin in bulk form is finely divided by means of an air micronizer.

The foregoing composition is useful for preventing or treating viral infection, or for treating a neoplastic disease, by the inhalation of 30 to 75 mg one to four times per day.

Example 13
Hard Gelatin Capsules

One thousand two-piece hard gelatin capsules for oral use, each capsule containing 100 mg of a didemnin or a nordidemnin.

The didemnin is finely divided by means of an air micronizer and encapsulated in the usual manner.

The foregoing capsules are useful for preventing or treating viral infection, or for treating a neoplastic disease, by the oral administration of one or two capsules one to four times a day.

Using the procedure above, capsules are similarly prepared containing didemnin or a nordidemnin in 50, 250 and 500 mg amounts by substituting 50 g, 250 g and 500 g of a didemnin for the 100 g used above.

Data show that the tentative structures for didemnin A, B and C can be shown as follows:

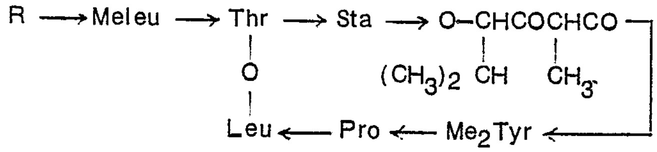

Didemnin A: $R = H$

Didemnin B: $R = CH_3CHOHCO \rightarrow Pro \rightarrow$

Didemnin C: $R = CH_3CHOHCO \rightarrow$

Where Meleu =

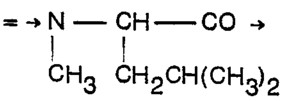

Thr =

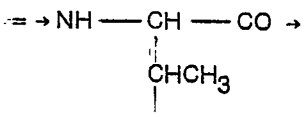

Sta =

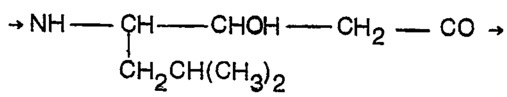

$Me_2Tyr$ =

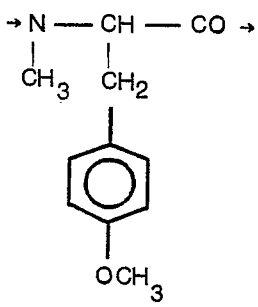

Pro =

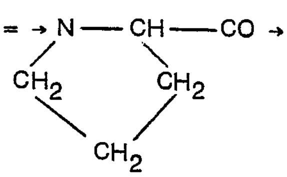

Leu =

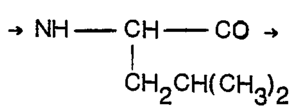

## Claims

1. An antibiotic (didemnin A) which is active against various DNA and RNA viruses, and which has the following characteristics:
(a) molecular weight of 942.5678 (mass spectroscopy);
(b) molecular formula —$C_{49}H_{78}N_6O_{12}$;
(c) soluble in methanol, ethanol, isopropanol, dioxane, ethyl acetate and chloroform, sparingly soluble in toluene, and insoluble in water;
(d) a characteristic infrared absorption spectrum as shown in Figure 1 of the drawings;
(e) amino acid composition as follows: one mole of leucine, N-methylleucine, threonine, proline, N,O-dimethyltyrosine and statine; and
(f) Rf of 0.46—0.52 (g:1 $CHCl_3$:MeOH as eluent);
or a salt thereof.

2. An antibiotic (didemnin B) which is active against various DNA and RNA viruses, and which has the following characteristics:
(a) molecular weight of 1111.6442 (mass spectroscopy);
(b) molecular formula —$C_{57}H_{89}N_7O_{15}$;
(c) soluble in methanol, ethanol, isopropanol, dioxane, ethyl acetate and chloroform, sparingly soluble in toluene, and insoluble in water;
(d) a characteristic infrared absorption spectrum as shown in Figure 2 of the drawings;
(e) amino acid composition as follows: one mole of leucine, N-methylleucine, threonine, N,O-dimethyltyrosine, statine and lactic acid, and two moles of proline; and
(f) Rf of 0.59—0.64 (9:1 $CHCl_3$:MeOH as eluent);

3. An antibiotic (didemnin C) which is active against various DNA and RNA viruses, and which has the following characteristics:
(a) molecular weight of 1013.5873 (mass spectroscopy);
(b) molecular formula —$C_{52}H_{81}N_6O_{14}$;
(c) soluble in methanol, ethanol, isopropanol, dioxane, ethyl acetate and chloroform, sparingly soluble in toluene, and insoluble in water;
(d) mass spectrum, obtained by electron impact, gives ions at *m/z:* 958, 887, 859, 842, 693, 675, 578, 547, 536, 494, 368, 283, 262, 237, 224, 200, 172, 154, 121 and 100;
(e) Rf of 0.66—0.72 (9:1 $CHCl_3$:MeOH as eluent); and
(f) amino acid composition as follows: one mole of leucine, N-methylleucine, threonine, proline, N,O-dimethyltyrosine, statine and lactic acid;
or a salt thereof.

4. An antibiotic (nordidemnin A) which is active against various DNA and RNA viruses, and which has the following characteristics:
(a) molecular weight of 928 (mass spectroscopy);
(b) molecular formula —$C_{48}H_{76}N_6O_{12}$;
(c) soluble in methanol, ethanol, isopropanol, dioxane, ethyl acetate and chloroform, sparingly soluble in toluene, and insoluble in water; and
(d) amino acid composition as follows: one mole of leucine, N-methylleucine; threonine, proline, N,O-dimethyltyrosine and norstatine;
or a salt thereof.

5. An antibiotic (nordidemnin B) which is active against various DNA and RNA viruses, and which has the following characteristics:
(a) molar weight of 1097 (mass spectroscopy);
(b) molecular formula —$C_{56}H_{87}N_7O_{15}$;
(c) soluble in methanol, ethanol, isopropanol, dioxane, ethyl acetate and chloroform, sparingly soluble in toluene, and insoluble in water; and
(d) amino acid composition as follows: one mole of leucine, N-methylleucine, threonine, N,O-dimethyltyrosine, norstatine and lactic acid, and two moles of proline;
or a salt thereof.

6. An antibiotic (nordidemnin C) which is active against various DNA and RNA viruses, and which has the following characteristics:
(a) molecular weight of 1000 (mass spectroscopy);
(b) molecular formula —$C_{51}H_{79}N_6O_{14}$;
(c) soluble in methanol, ethanol, isopropanol, dioxane, ethyl acetate and chloroform, sparingly soluble in toluene, and insoluble in water; and
(d) amino acid composition as follows: one mole of leucine, N-methylleucine, threonine, proline, N,O-dimethyltyrosine, norstatine and lactic acid;
or a salt thereof.

7. An acylate of an antibiotic as claimed in any preceding claim, wherein the acyl radical is ($C_{1-8}$ alkoxy)-carbonyl or optionally substituted hydrocarbon $O_{2-18}$ acyl in which any substituent is selected from halogen, nitro, hydroxy, amino, cyano, thiocyano and $C_{1-8}$ alkoxy.

8. A process for preparing an antibiotic as claimed in any of claims 1 to 3, which comprises
(a) extracting a suitable marine tunicate of the family Didemnidae with MeOH:toluene (3:1 v/v);
(b) subjecting the resulting extract to silica gel chromatography using the solvent system $CHCl_3$:MeOH in step gradient fashion; and
(c) recovering the antibiotic.

9. A process for preparing an antibiotic as claimed in any of claims 4 to 6, which comprises
(a) extracting a suitable marine tunicate of the family Didemnidae with MeOH:toluene (3:1 v/v);
(b) subjecting the resulting extract to silica gel chromatography using the solvent system $CHCl_3$:MeOH in step gradient fashion; and
(c) recovering the antibiotic by reverse phase high pressure liquid chromatography.

10. A process according to claim 8 or claim 9, wherein the tunicate is of the family Didemnidae, *Trididemnum sp.*

11. A pharmaceutical composition comprising an antibiotic, or an acylate thereof, as claimed in any of claims 1 to 7, in association with a physiologically acceptable excipient.

## Revendications

1. Antibiotique (didemnine A) qui est actif contre divers virus d'ADN et d'ARN et qui a les caractéristiques suivantes:
(a) poids moléculaire de 942,5678 (spectroscopie de masse);
(b) formule moléculaire —$C_{49}H_{78}N_6O_{12}$;
(c) soluble dans le méthanol, l'éthanol, l'isopropanol, le dioxanne, l'acétate d'éthyle et le chloroforme, peu soluble dans le toluène et insoluble dans l'eau;
(d) spectre d'absorption infrarouge caractéristique tel que représenté sur la figure 1 des dessins;
(e) composition en amino-acides suivante: une mole de leucine, N-méthyl-leucine, thréonine, proline, N,O-diméthyltyrosine et statine; et
(f) Rf de 0,46—0,52 ($CHCl_3$:MeOH à 9:1 comme éluant);
ou un sel de cet antibiotique.

2. Antibiotique (didemnine B) qui est actif contre divers virus d'ADN et d'ARN et qui a les caractéristiques suivantes:
(a) poids moléculaire de 1111,6442 (spectroscopie de masse);
(b) formule moléculaire —$C_{57}H_{89}N_7O_{15}$;
(c) soluble dans le méthanol, l'éthanol, l'isopropanol, le dioxanne, l'acétate d'éthyle et le chloroforme, peu soluble dans le toluène et insoluble dans l'eau;
(d) spectre d'absorption infrarouge caractéristique tel que représenté sur la figure 2 des dessins;
(e) composition en amino-acides suivante: une mole de leucine, N-méthyl-leucine, thréonine, N,O-diméthyltyrosine, statine et acide lactique et 2 moles de proline; et
(f) Rf de 0,59—0,64 ($CHCl_3$:MeOH à 9:1 comme éluant);
ou un sel de cet antibiotique.

3. Antibiotique (didemnine C) qui est actif contre divers virus d'ADN et d'ARN et qui a les caractéristiques suivantes:
(a) poids moléculaire 1013,5873 (spectroscopie de masse);
(b) formule moléculaire —$C_{52}H_{81}N_6O_{14}$;
(c) soluble dans le méthanol, l'éthanol, l'isopropanol, le dioxanne, l'acétate d'éthyle et le chloroforme, peu soluble dans le toluène et insoluble dans l'eau;
(d) spectre de masse, obtenu par chocs d'électrons, donnant des ions à m/z: 958, 887, 859, 842, 693, 675, 578, 547, 536, 494, 368, 283, 262, 237, 224, 200, 172, 154, 121 et 100;
(e) Rf de 0,66—0,72 ($CHCl_3$:MeOH à 9:1 comme éluant); et
(f) composition en amino-acides suivantes: une mole de leucine, N-méthyl-leucine, thréonine, proline, N,O-diméthyltyrosine, statine et acide lactique;
ou un sel de cet antibiotique.

4. Antibiotique (nordidemnine A) qui est actif contre divers virus d'ADN et d'ARN et qui présente les caractéristiques suivantes:
(a) poids moléculaire 928 (spectroscopie de masse);
(b) formule moléculaire —$C_{48}H_{76}N_6O_{12}$;
(c) soluble dans le méthanol, l'éthanol, l'isopropanol, le dioxanne, l'acétate d'éthyle et le chloroforme, peu soluble dans le toluène et insoluble dans l'eau; et
(d) composition en amino-acides suivante: une mole de leucine, N-méthyl-leucine, thréonine, proline, N,O-diméthyltyrosine et norstatine;
ou un sel de cet antibiotique.

5. Antibiotique (nordidemnine B) qui est actif contre divers virus d'ADN et d'ARN et qui présente les caractéristiques suivantes:
(a) poids moléculaire 1097 (spectroscopie de masse);
(b) formule moléculaire —$C_{56}H_{87}N_7O_{15}$;

(c) soluble dans le méthanol, l'éthanol, l'isopropanol, le dioxanne, l'acétate d'éthyle et le chloroforme, peu soluble dans le toluène et insoluble dans l'eau; et

(d) composition en amino-acides suivante: une mole de leucine, N-méthyl-leucine, thréonine, N,O-diméthyltyrosine, norstatine et acide lactique et 2 moles de proline;

ou un sel de cet antibiotique.

6. Antibiotique (nordidemnine C) qui est actif contre divers virus d'ADN et d'ARN et qui a les caractéristiques suivantes:

(a) poids moléculaire 1000 (spectroscopie de masse);

(b) formule moléculaire —$C_{51}H_{79}N_6O_{14}$;

(c) soluble dans le méthanol, l'éthanol, l'isopropanol, le dioxanne, l'acétate d'éthyle et le chloroforme, peu soluble dans le toluène et insoluble dans l'eau;

(d) composition en amino-acides suivante: une mole de leucine, N-méthyl-leucine, thréonine, proline, N,O-diméthyltyrosine, norstatine et acide lactique;

ou un sel de cet antibiotique.

7. Acylate d'un antibiotique suivant l'une quelconque des revendications précédentes, dans lequel le radical acyle est un radical (alkoxy en $C_1$ à $C_8$)-carbonyle ou un radical acyle hydrocarboné en $C_2$ à $C_{18}$ éventuellement substitué dans lequel tout substituant est choisi entre un halogène, un radical nitro, hydroxy, amino, cyano, thiocyano et alkoxy en $C_1$ à $C_8$.

8. Procédé de préparation d'un antibiotique suivant l'une quelconque des revendications 1 à 3, qui consiste

(a) à extraire un tunicier marin convenable de la famille des Didemnidae avec un mélange MeOH:toluène (3:1 volume/volume);

(b) à soumettre l'extrait résultant à une chromatographie sur gel de silice en utilisant comme système de solvants un mélange $CHCl_3$:MeOH par étapes selon un gradient; et

(c) à recueillir l'antibiotique.

9. Procédé de préparation d'un antibiotique suivant l'une quelconque des revendications 4 à 6, qui consiste

(a) à extraire un tunicier marin convenable de la famille des Didemnidae avec un mélange MeOH:toluène (3:1 volume/volume);

(b) à soumettre l'extrait résultant à une chromatographie sur gel de silice en utilisant le système de solvants $CHCl_3$:MeOH par étapes selon un gradient; et

(c) à recueillir l'antibiotique par chromatographie en phase liquide sous haute pression à phase inverse.

10. Procédé suivant la revendication 8 ou la revendication 9, dans lequel le tunicier est une espèce du genre *Tridemnum* de la famille des Didemnidae.

11. Composition pharmaceutique, comprenant un antibiotique ou son acylate suivant l'une quelconque des revendications 1 à 7 en association avec un excipient physiologiquement acceptable.

**Patentansprüche**

1. Antibiotikum (Didemnin A), das gegen verschiedenen DNA- und RNA-Viren aktiv ist und die folgenden Eigenschaften aufweist:

(a) Molekulargewicht 942,5678 (Massenspektroskopie);

(b) Bruttoformel $C_{49}H_{78}N_6O_{12}$;

(c) löslich in Methanol, Ethanol, Isopropanol, Dioxan, Ethylacetat und Chloroform, mässig löslich in Toluol und unlöslich in Wasser;

(d) charakteristisches IR-Spektrum, wie in Fig. 1 der Zeichnung gezeigt;

(e) folgende Aminosäure-Zusammensetzung: 1 Mol Leucin, N-Methylleucin, Threonin, Prolin, N,O-Dimethyltyrosin und Statin; und

(f) Rf-Wert von 0,46—0,52 (9:1 $CHCl_3$:MeOH als Eluiermittel);

oder ein Salz davon.

2. Antibiotikum (Didemnin B), das gegen verschiedene DNA- und RNA-Viren aktiv ist und die folgenden Eigenschaften aufweist:

(a) Molekulargewicht 1111,6442 (Massenspektroskopie);

(b) Bruttoformel $C_{57}H_{89}N_7O_{15}$;

(c) löslich in Methanol, Ethanol, Isopropanol, Dioxan, Ethylacetat und Chloroform, mässig löslich in Toluol und unlöslich in Wasser;

(d) charakteristisches IR-Spektrum, wie in Fig. 2 der Zeichnungen gezeigt;

(e) folgende Aminosäure-Zusammensetzung: 1 Mol Leucin, N-Methylleucin, Threonin, N,O-Dimethyltyrosin, Statin und Milchsäure und 2 Mole Leucin; und

(f) Rf-Wert von 0,59—0,64 (9:1 $CHCl_3$:MeOH als Eluiermittel;

oder ein Salz davon.

3. Antibiotikum (Didemnin C), das gegen verschiedene DNA- und RNA-Viren aktiv ist und die folgenden Eigenschaften aufweist:

(a) Molekulargewicht 1013,5873 (Massenspektroskopie);

(b) Bruttoformel $C_{52}H_{81}N_6O_{14}$;
(c) löslich in Methanol, Ethanol, Isopropanol, Dioxan, Ethylacetat und Chloroform, mässig löslich in Toluol und unlöslich in Wasser;
(d) Massenspektrum, erhalten durch Elektronenstoss, ergibt Ionen bei m/z: 958, 887, 859, 842, 693, 675, 578, 547, 536, 494, 368, 283, 262, 237, 224, 200, 172, 154, 121 und 100;
(e) Rf-Wert von 0,66—0,72 (9:1 $CHCl_3$:MeOH als Eluiermittel; und
(f) folgende Aminosäure-Zusammensetzung: 1 Mol Leucin, N-Methylleucin, Threonin, Prolin, N,O-Dimethyltyrosin, Statin und Milchsäure;
oder ein Salz davon.

4. Antibiotikum (Nordidemnin A), das gegen verschiedenen DNA- und RNA-Viren aktiv ist und die folgenden Eigenschaften aufweist:
(a) Molekulargewicht 928 (Massenspektroskopie);
(b) Bruttoformel $C_{48}H_{76}N_6O_{12}$;
(c) löslich in Methanol, Ethanol, Isopropanol, Dioxan, Ethylacetat und Chloroform, mässig löslich in Toluol und unlöslich in Wasser; und
(d) folgende Aminosäure-Zusammensetzung: 1 Mol Leucin, N-Methylleucin, Threonin, Prolin, N,O-Dimethyltyrosin und Norstatin;
oder ein Salz davon.

5. Antibiotikum (Nordidemnin B), das gegen verschiedenen DNA- und RNA-Viren aktiv ist und die folgenden Eigenschaften aufweist:
(a) Molekulargewicht 1097 (Massenspektroskopie);
(b) Bruttoformel $C_{56}H_{87}N_7O_{15}$;
(c) löslich in Methanol, Ethanol, Isopropanol, Dioxan, Ethylacetat und Chloroform, mässig löslich in Toluol und unlöslich in Wasser; und
(d) folgende Aminosäure-Zusammensetzung: 1 Mol Leucin, N-Methylleucin, Threonin, N,O-Dimethyltyrosin, Norstatin und Milchsäure und 2 Mol Prolin;
oder ein Salz davon.

6. Antibiotikum (Nordidemnin C), das gegen verschiedenen DNA- und RNA-Viren aktiv ist und die folgenden Eigenschaften aufweist:
(a) Molekulargewicht 1000 (Massenspektroskopie);
(b) Bruttoformel $C_{51}H_{79}N_6O_{14}$;
(c) löslich in Methanol, Ethanol, Isopropanol, Dioxan, Ethylacetat und Chloroform, mässig löslich in Toluol und unlöslich in Wasser; und
(d) foldende Aminosäure-Zusammensetzung: 1 Mol Leucin, N-Methylleucin, Threonin, Prolin, N,O-Dimethyltyrosin, Norstatin und Milchsäure;
oder ein Salz davon.

7. Acylat eines Antibiotikums gemäss einem der vorhergehenden Ansprüche, worin der Acylrest ($C_1$—$C_8$-Alkoxy)-carbonyl oder gegebenenfalls substituiertes Kohlenwasserstoff-$C_2$—$C_{18}$-Acyl bedeutet und worin die Substituenten aus der Gruppe Halogen, Nitro, Hydroxy, Amino, Cyano, Thiocyano und $C_1$—$C_8$-Alkoxy ausgewählt sind.

8. Verfahren zur Herstellung eines Antibiotikums gemäss einem der Ansprüche 1—3, dadurch gekennzeichnet, dass man
(a) ein geeignetes Meer-Manteltier der Familie Didemnidae mit MeOH:Toluol (3:1 v/v) extrahiert;
(b) den erhaltenen Extrakt der Chromatographie an Silicagel unter Verwendung des Lösungsmittelsystems $CHCl_3$:MeOH auf Stufengradienten-Weise unterwirft; und
(c) das Antibiotikum gewinnt.

9. Verfahren zur Herstellung eines Antibiotikums gemäss einem der Ansprüche 4—6, dadurch gekennzeichnet, dass man
(a) ein geeignetes Meer-Manteltier der Familie Didemnidae mit MeOH:Toluol (3:1 v/v) extrahiert;
(b) den erhaltenen Extrakt der Chromatographie an Silicagel unter Verwendung des Lösungsmittelsystems $CHCl_3$:MeOH auf Stufengradienten-Weise unterwirft; und
(c) das Antibiotikum durch Hochdruck-Flüssigkeits-Chromatographie mit umgekehrter Phase gewinnt.

10. Verfahren nach Anspruch 8 oder 9, dadurch gekennzeichnet, dass das Manteltier aus der Familie Didemnidae, Trididemnum sp. stammt.

11. Pharmazeutische Zusammensetzung, dadurch gekennzeichnet, dass sie ein Antibiotikum oder ein Acylat davon gemäss einem der Ansprüche 1—7 zusammen mit einem physiologisch annehmbaren Arzneiträgermaterial enthält.

FIG. I

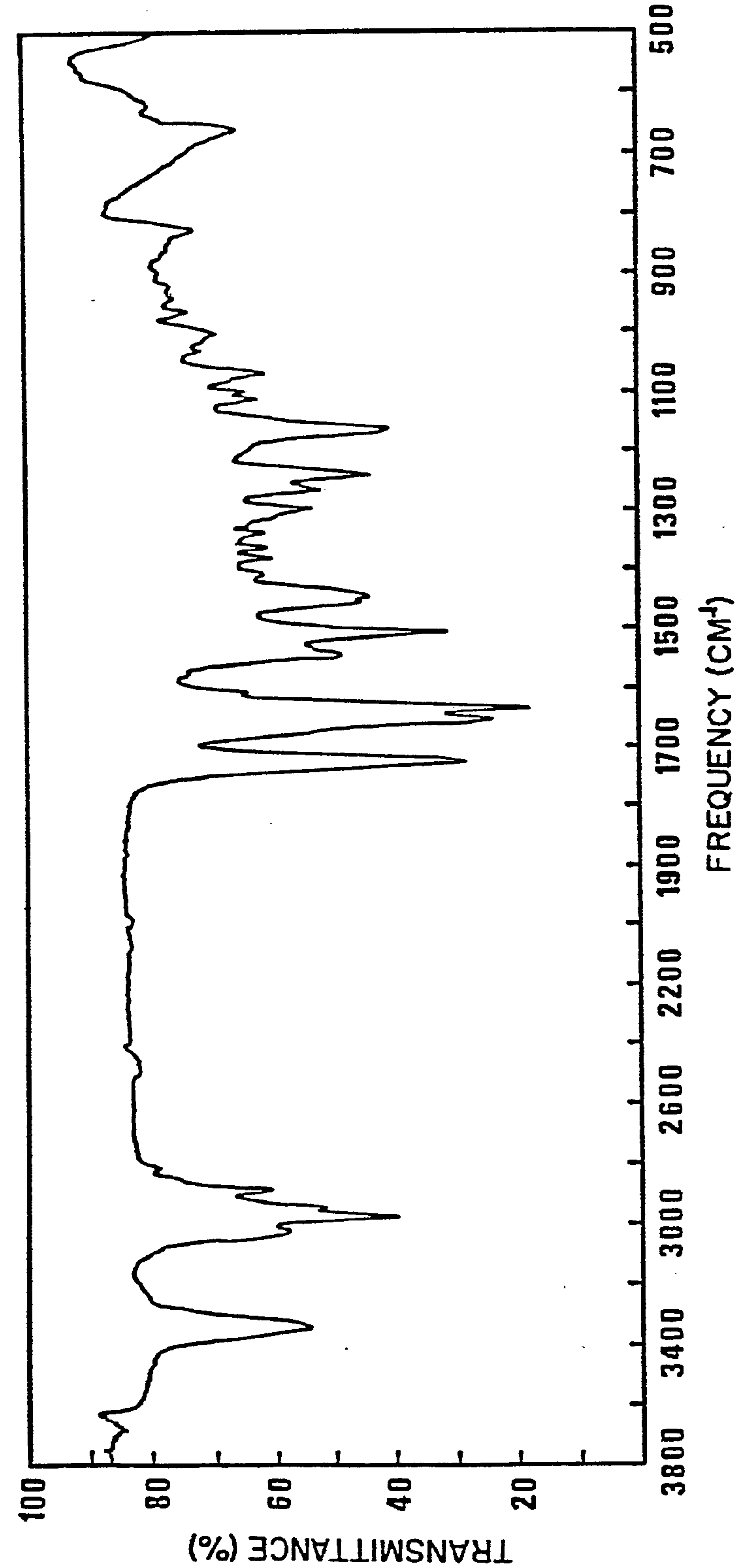
TRANSMITTANCE (%)
100
80
60
40
20
FREQUENCY (CM⁻¹)
3800
3400
3000
2600
2200
1900
1700
1500
1300
1100
900
700
500

FIG. 2

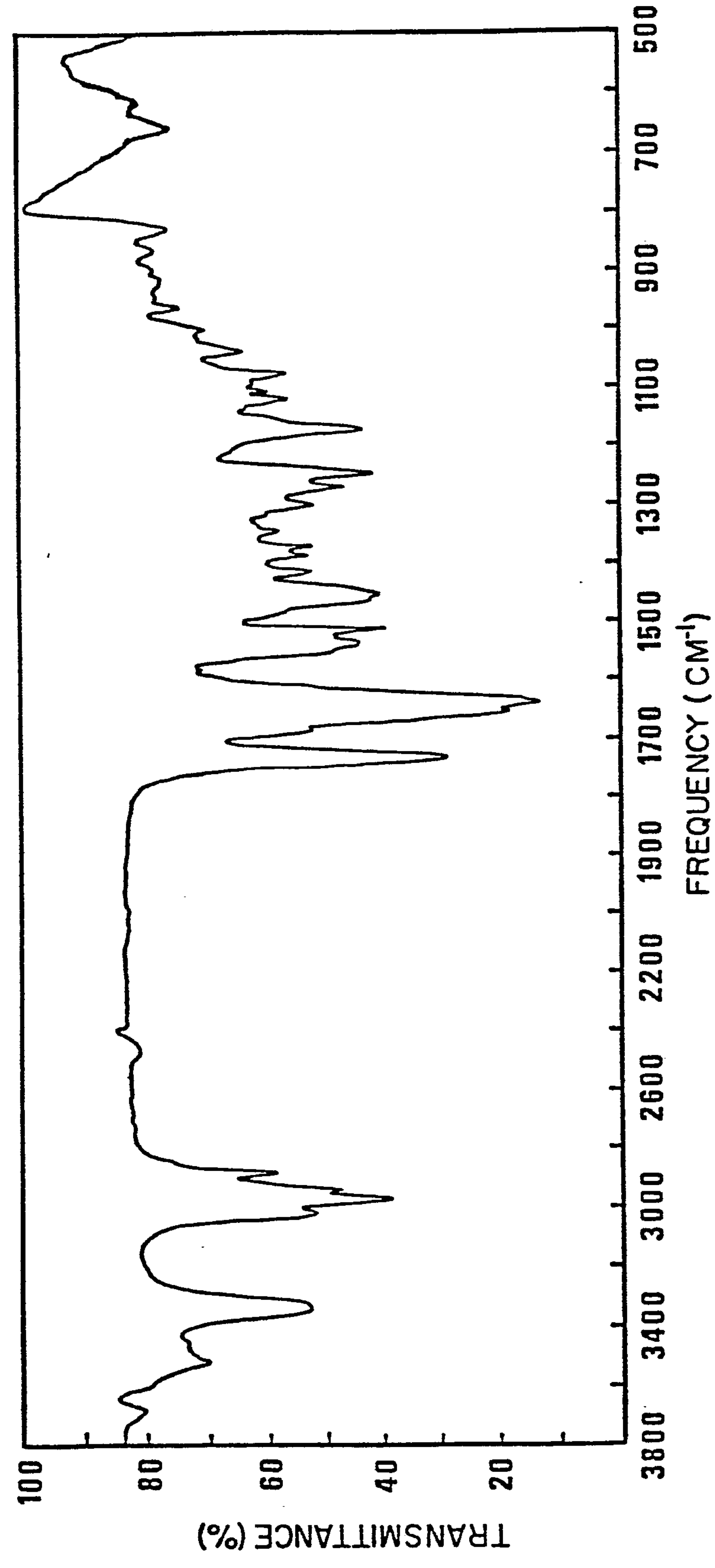
TRANSMITTANCE (%)
100
80
60
40
20
FREQUENCY (CM-1)
3800
3400
3000
2600
2200
1900
1700
1500
1300
1100
900
700
500